# EUROPEAN PATENT SPECIFICATION

(11) **EP 0 261 802 B1**
(45) Date of publication and mention of the grant of the patent: **08.01.1997**
(21) Application number: 87307490.0
(22) Date of filing: 25.08.1987
(51) Int. Cl.: C07D 487/18, C07C 23/38, A61K 31/53, A61K 31/025, A61K 31/66, C07C 23/46, C07D 321/00, C07D 245/04, C07C 23/20, C07C 55/02, C07C 211/16

(54) **Perfluoro hexamethyltetramine and synthetic phospholipids, useful in synthetic blood**
Perfluoro-hexamethyltetramin und synthetische Phopholipide, verwendbar als synthetisches Blut
Perfluoro hexaméthyltétramine et phospholipides synthétiques utiles pour sang synthétique

(30) Priority: 25.08.1986 US 899903
(43) Date of publication of application: 30.03.1988
(73) Proprietor: INTERNATIONAL THERAPEUTICS INC., Pasadena California 91103 (US)
(72) Inventor: Dandliker, Walter B., La Jolla California 92037 (US); Watson, Keith W.R., Alpine California 92001 (US); Drees, Thomas C., Flintridge California 91011 (US)
(74) Representative: Holdcroft, James Gerald, Dr.

(56) References cited:
- US-A- 4 105 798
- US-A- 4 402 984
- ANGEWANDTE CHEMIE, vol. 90, 1978, Verlag Chemie Weinheim- New York JEAN G. RIESS UND MAURICE LE BLANC "Perfluor-Verbindungen als Blutersatzmittel" pages 654-668
- CHEMICAL ABSTRACTS, vol. 98, no. 22, May 30, 1983, Columbus, Ohio, USA R.E. MOORE "Synthesis and physical properties of perfluoro-compounds useful as synthetic blood candidates" page 369, column 1, Abstract-no. 185 511g & Oxugen Carrying Colloidal Blood Substitutes, Int. Symp. Perfluorochem. Blood Substitutes, 5th 1981 (Pub. 1982)

## Description

This invention relates generally to synthetic blood, and more particularly to an improved blood substitute offering improvements in oxygen carrying capacity and stability, as well as lessened risk of anaphylactoid reaction.

The facile transport of oxygen through Teflon (polyperfluoroethylene) membranes has been well known for many years. The realization of the compatibility of perfluorocarbons with oxygen led to a series of research efforts which subsequently arrived at the utilization of perfluorochemicals as oxygen carriers in a new generation of blood substitutes.

Initial work by Leland Clark of Cincinnati Childrens Hospital, Robert Geyer of Harvard and Henry Sloviter of the University of Pennsylvania, continued and extended by Naito and co-workers, led to a preparation of perfluorodecalin (Fluosol DA 20%) produced for clinical testing by Green Cross of Osaka, Japan. Fluosol DA functioned as an oxygen carrier in animal experiments and showed considerable promise for human use.

However, Fluosol DA had several significant drawbacks. First, the emulsion of fluorochemical droplets in an aqueous phase was inherently unstable, both thermodynamically and kinetically, necessitating storage of the emulsion in the frozen state. This instability also entailed a laborious and time consuming blending of the emulsion with other accessory solutions immediately before use. A second major problem with Fluosol DA was the necessity of maintaining the patient on 70 to 100% oxygen to ensure sufficient oxygen supply and exchange in the tissues. Finally, limited clinical experience with Fluosol DA showed an incidence of transfusion reactions and, in order to avoid this problem, led to the pretreatment of patients with steroids in the event a small test dose indicated sensitivity; this type of sensitivity appeared in 3% or less of all cases.

Allegedly improved blood substitutes are disclosed in US-A-4,105,798, which are based on non-aromatizable perfluorinated polycyclic hydrocarbons having 9-18 carbon atoms and at least two bridgehead carbon atoms linked through a bridge containing at least one C atom.

### SUMMARY OF THE INVENTION

It is a major object of the invention to provide an improved blood substitute, employing

perfluorochemicals capable in the presence of suitable emulsifying agents of forming emulsions stable at room temperature and possessing enhanced oxygen carrying capacity. It is an additional object of the invention to overcome the toxic (anaphylactoid) reaction problem by the use of synthetic phospholipids in the substitute blood in which such perfluorochemicals are employed.

### DETAILED DESCRIPTION

The solubility of oxygen in fluorochemicals is correlated with the isothermal compressibility of the liquid fluorochemical. The oxygen molecules pack into voids or cavities in the liquid structure in the process of solution, but do not interact significantly with the fluorochemical molecules as evidenced by the quite small enthalpies of solution. In the perfluoro hexamethylenetetramine of this invention, the presence of voids or cavities has been intentionally incorporated into the molecular structure. Thanks to the molecular structure, the voids or pockets accommodate oxygen molecules in the interstices of individual molecules.

The perfluorocompounds referred to above have structures indicated by the formula (1) given below.

As an illustration, formula (2) shows the totally fluorinated form of hexamethylenetetramine according to this invention, formula (1), but with O₂ molecules in structure voids, and it will be understood the O₂ molecules can be transported by the perfluoro compound and also within voids or interstices formed by close packing of the structure (2). 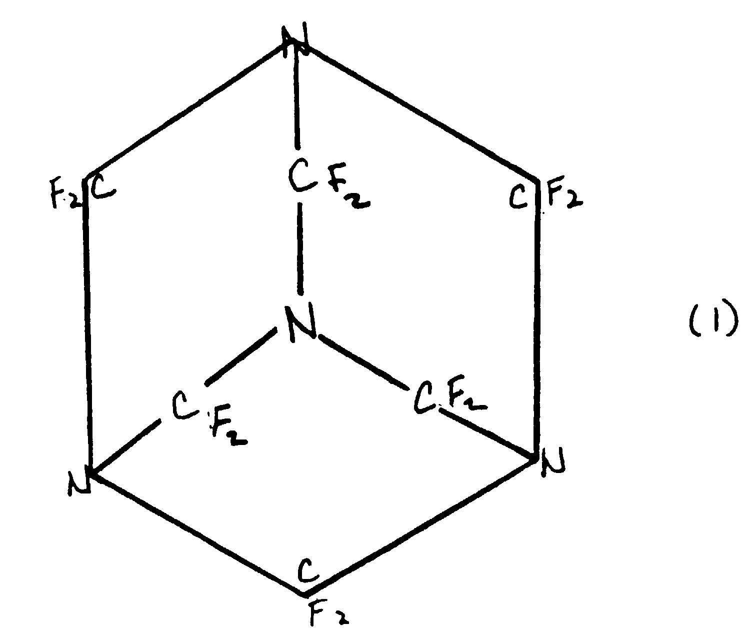 (1) Skeleton formula of perfluoro form of hexamethylenetetramine. 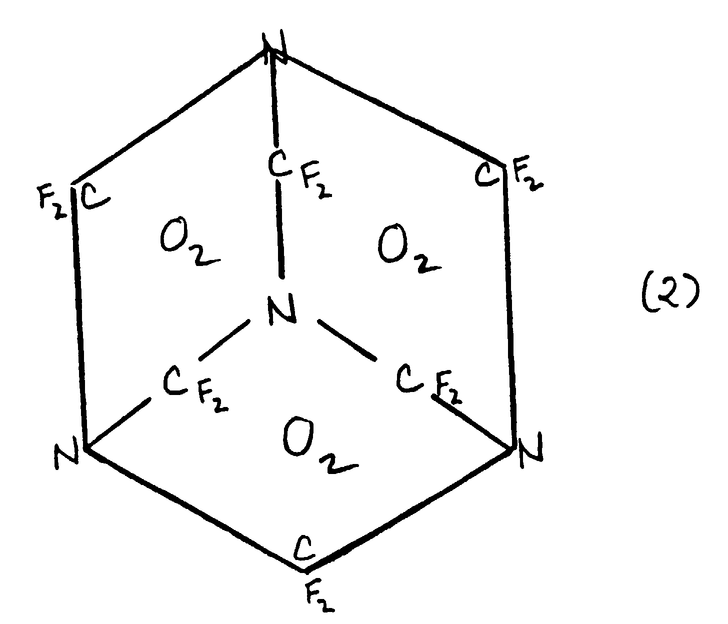 (2) Same as (1), but transporting O₂.

The compounds of this invention, e.g. in admixture with appropriate surfactants, when emulsified in water along with electrolytes and colloids compatible with natural blood, typically produce droplets which are suspended in solution and which are storable and stable at room temperature, the solution then being directly usable as an oxygen carrying blood substitute. O₂ molecules are easily loosely retained for transport in the molecules, as indicated in (2) above.

The emulsion contains a non-toxic fraction derived from Pluronic F-68 or equivalent, together with one or more synthetic phospholipids as emulsifiers or surfactants to stabilize the emulsion. The fraction from Pluronic F-68 is prepared by fractional precipitation with organic solvents or salts or by absorption or partition chromatography, starting in either case with commercially available Pluronic F-68. Pluronic F-68* is not a uniform molecular species but instead consists of a mixture of molecules of differing molecular weight. The effectiveness of these different molecular species as emulsifying agents is a function of molecular weight or chain length. It is for this reason that when applied to this invention, highly refined fractions of optimal molecular weight are used in making the fluorochemical emulsion. In addition, the fractionation employed to prepare these purified materials tends to remove any residual materials toxic to humans or deleterious to red cells. The synthetic phospholipids differ from one another as to whether the overall structure corresponds to that of a lecithin, cephalin, plasmalogen or sphingomyelin and in the nature of the fatty acid side chains in the structure. The fatty acids differ in the number of carbon atoms, the number and placement of double bonds and in the presence or absence of alicyclic, aromatic or heterocyclic rings. Synthetic phospholipids, unlike yolk phospholipids contain no trace of egg proteins which in many individuals are highly allergenic. The structure of a typical lecithin is as follows: 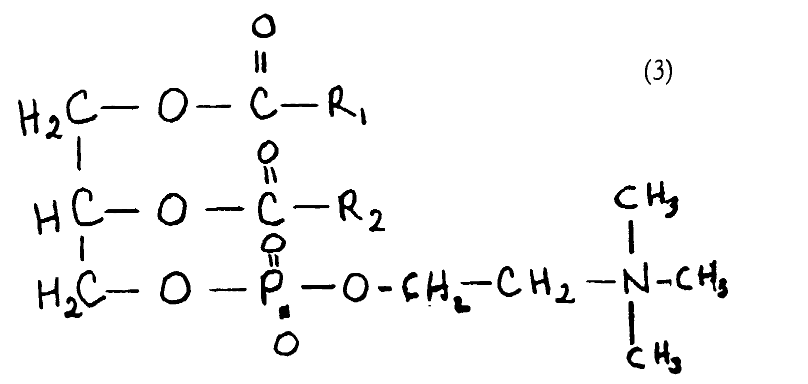 where R₁ and R₂ are fatty acids selected from the group stearic acid, linoleic acid, eicosapentaenoic acid and dogosaheyaenoic acid.
* polyoxyperopylene-polyoxyethylene block co-polymer

In preparing and storing the fluorochemical emulsions of this invention it is essential to prevent degradative reactions involving any of the components. If such reactions are allowed to occur, emulsion instability and/or toxicity may result.

Several types of such reactions are either known to occur, or may be logically expected to occur, if proper preventive measures are ignored. First, certain fluorochemicals, under the energetic influence of homogenization or sonication, especially in the presence of oxygen, can degrade to yield fluoride ion which is quite toxic. Second, any unsaturation in the fatty acid side chains of the phospholipid emulsifiers may result in the formation of peroxides if oxygen is present and if such reactions are not inhibited. For these reasons, oxygen is excluded and, in addition, antioxidants such as vitamin E or other tocopherols are added to provide stabilization for oxygen-labile components.

An emulsion embodying the present invention, prepared for intravenous administration, and also containing a synthetic phospholipid, is as follows:

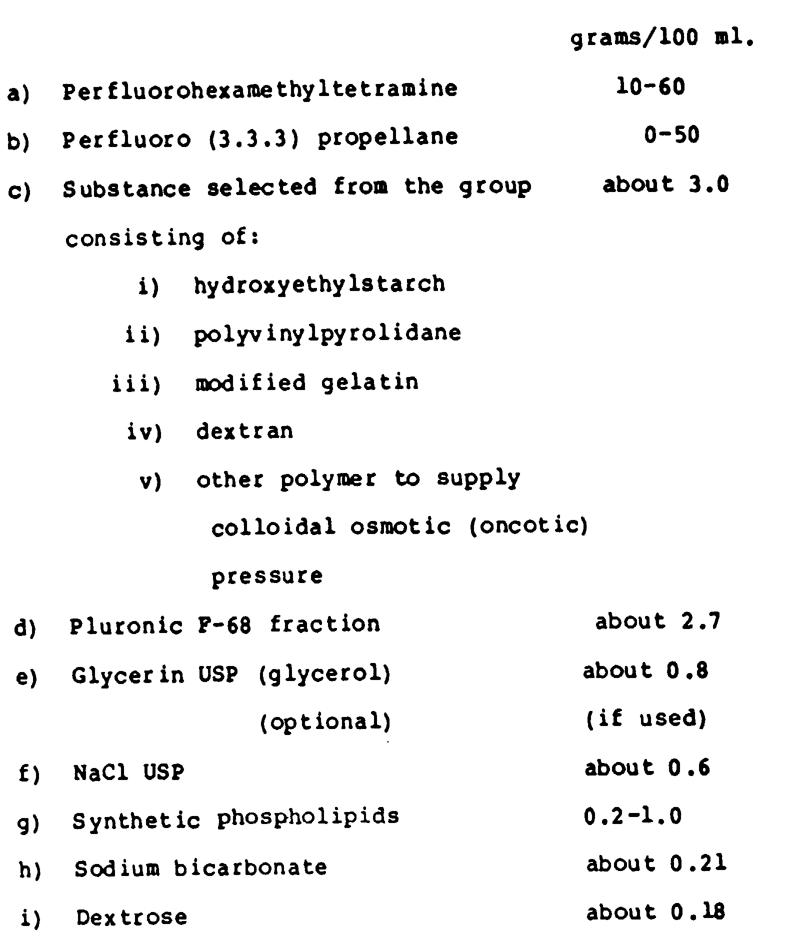

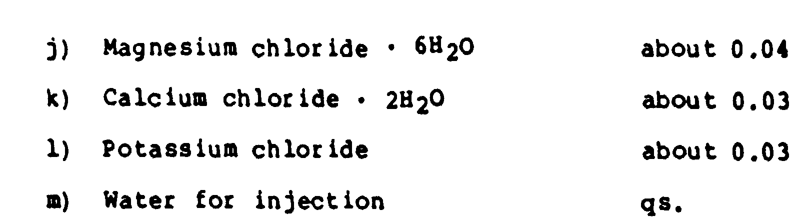

The following are specific examples, with constituents the same as listed above in a)--m):

| | Examples (gms/100 ml.) | | | | | |
|---|---|---|---|---|---|---|
| Constituents | 1 | 2 | 3 | 4 | 5 | 6 |
| a) | 20 | 20 | 25 | 25 | 30 | 30 |
| b) | 40 | 40 | 35 | 35 | 30 | 30 |
| c) | 3.0 | 3.0 | 3.0 | 3.0 | 3.0 | 3.0 |
| d) | 2.7 | 2.7 | 2.7 | 2.7 | 2.7 | 2.7 |
| e) | 0.8 | 0. | 0.8 | 0. | 0.8 | 0. |
| f) | 0.6 | 0.6 | 0.6 | 0.6 | 0.6 | 0.6 |
| g) | 0.4 | 0.4 | 0.4 | 0.4 | 0.4 | 0.4 |
| h) | 0.21 | 0.21 | 0.21 | 0.21 | 0.21 | 0.21 |
| i) | 0.18 | 0.18 | 0.18 | 0.18 | 0.18 | 0.18 |
| j) | 0.043 | 0.043 | 0.043 | 0.043 | 0.043 | 0.043 |
| k) | 0.036 | 0.036 | 0.036 | 0.036 | 0.036 | 0.036 |
| l) | 0.034 | 0.034 | 0.034 | 0.034 | 0.034 | 0.034 |
| m) | qs | qs | qs | qs | qs | qs |

In the above, the synthetic phospholipids are of the structure (3) above.

Preferably, the emulsion is prepared under nitrogen or a noble gas to protect labile components of the system from oxidative degradation, and the final product is packaged under nitrogen or a noble gas to protect the product from oxidation during storage.

Addition to the product, of vitamin E, mixed tocopherols or other antioxidants compatible with the product and with red cells, further protects labile components of the mixture against oxidation.

Fractions of Pluronic F-68 are selected for their superior ability to form and to stabilize emulsions of perfluorochemicals in aqueous solutions compatible with blood.

Oxygen carriage or transport occurs in two ways, i.e. in the molecule (see position of O₂ in molecular form (3); and O₂ entrapment between the molecules.

Consider the following diagram for example, wherein the perfluoro molecules are denoted by large circles, moving in a capillary, and the oxygen molecules are denoted by dots in the interstices between the large molecules. (Also note the oxygen molecules within the circles, i.e. the first-way of O₂ transport referred to above).

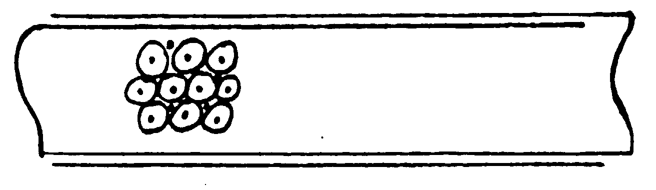

Advantageous results includes greater O₂ transport, whereby in-breathing of excessive oxygen by the patient is not required --i.e. the patient can breath ordinary air, exclusively.

## Claims (Claims for the following Contracting State(s): BE, CH, DE, FR, GB, IT, LI, LU, NL, SE)

1. The compound perfluoro hexamethylenetetramine.

2. The compound of claim 1 having the following structure:

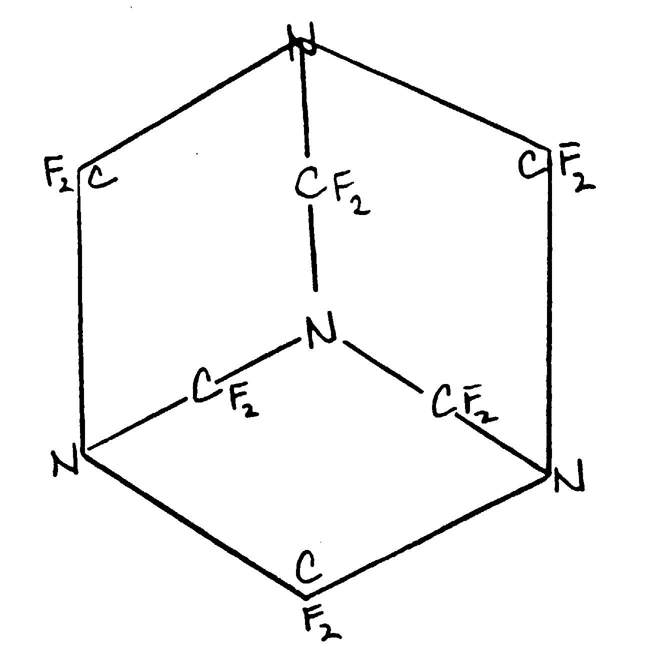

3. The compound of claim 2, wherein O₂ is trapped in the molecular structure and having the following structure:

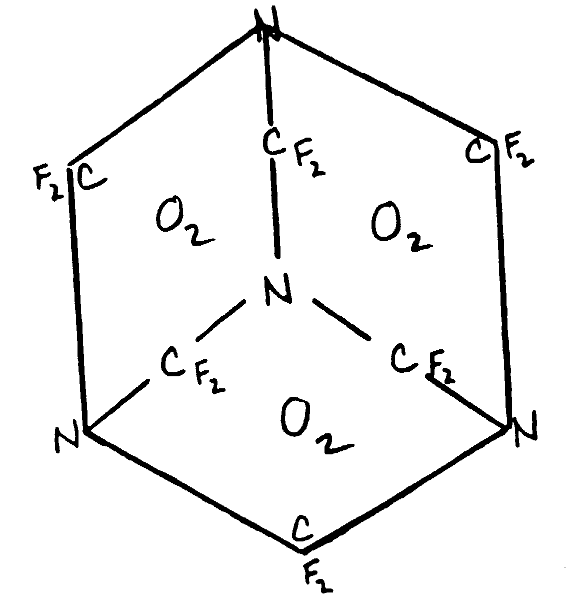

4. An artificial blood comprising an emulsion of the compound of any one of claims 1 to 3, in water, said emulsion containing a non-toxic emulsifier and a synthetic phospholipid which contains no trace of egg proteins.

5. The artificial blood of claim 4, wherein the synthetic phospholipid has the following structure: 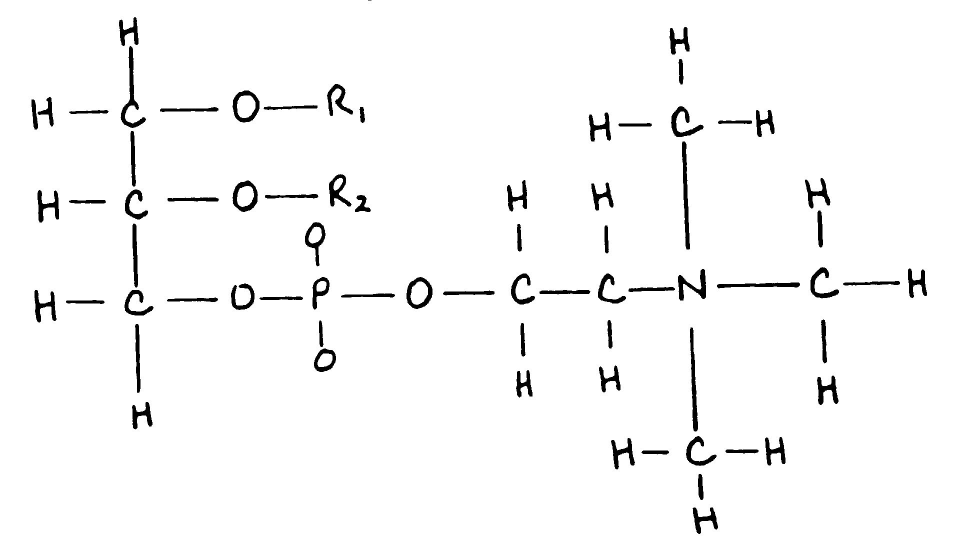 where R₁ and R₂ are fatty acids.

6. A synthetic blood having the following composition:

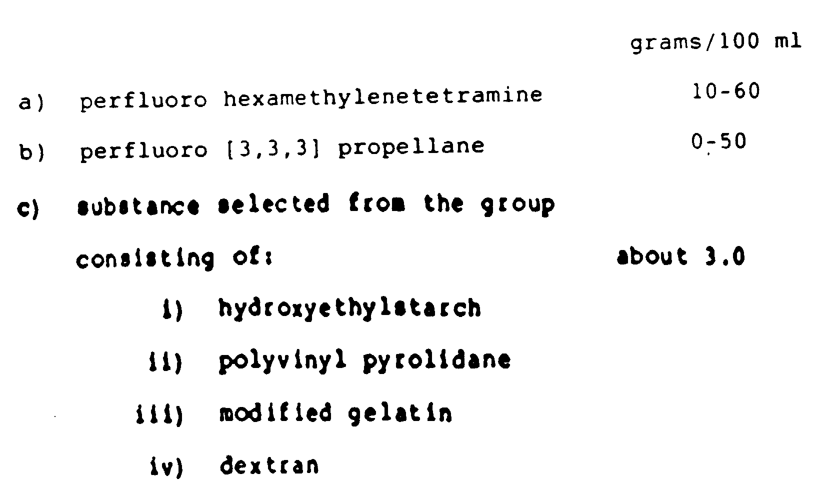

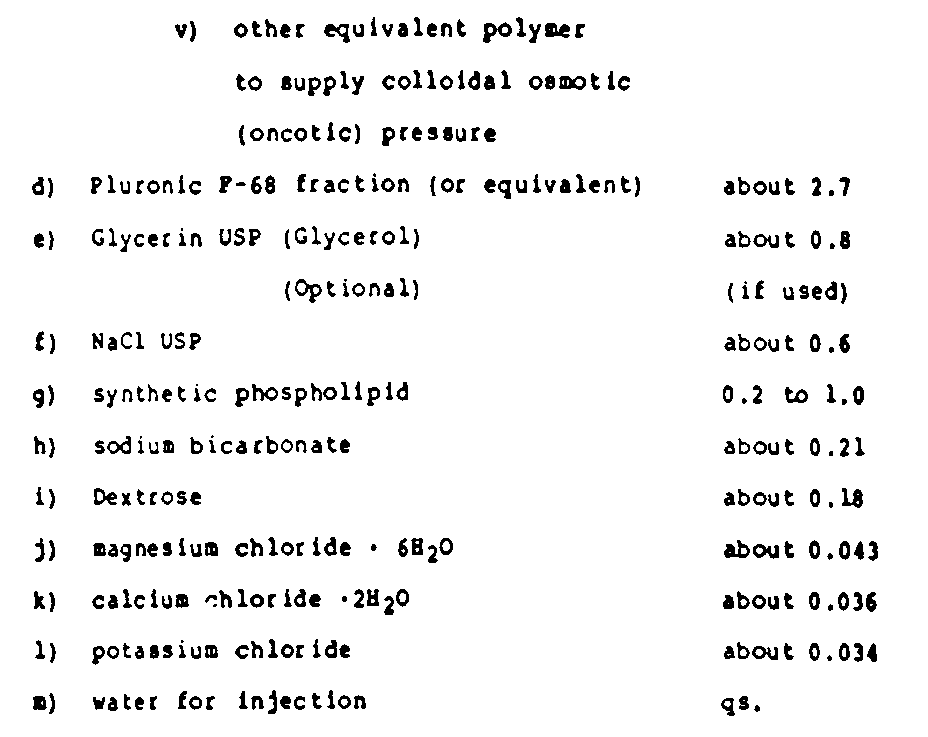

7. Material according to any one of claims 1 to 6, for use in therapy.

8. Use of material according to any one of claims 1 to 6 for the manufacture of a medicament for use as an artificial blood.

## Claims (Claims for the following Contracting State(s): AT, ES, GR)

1. A method of making an artificial blood, comprising making an emulsion in water, said emulsion containing the compound perfluoro hexamethylenetetramine.

2. A method according to claim 1 wherein the compound has the following structure:

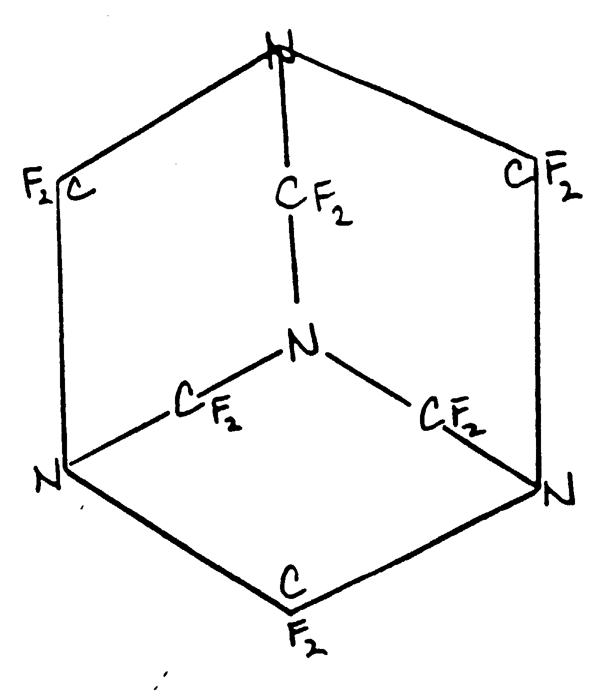

3. A method according to claim 2, wherein O₂ is trapped in the molecular structure of the compound, as follows:

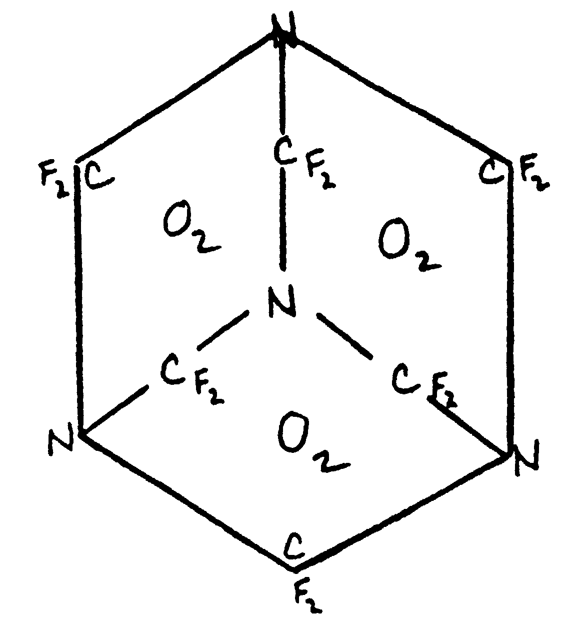

4. A method according to any one of claims 1 to 3, wherein a non-toxic emulsifier and a synthetic phospholipid which contains no trace of egg proteins is included in the emulsion.

5. A method according to claim 4, wherein the synthetic phospholipid has the following structure: 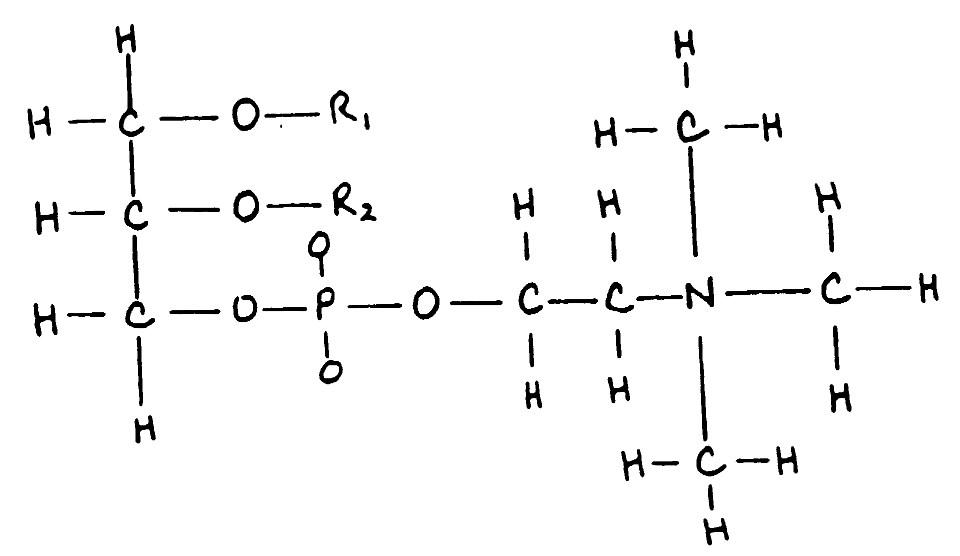 where R₁ and R₂ are fatty acids.

6. A method according to claim 1, comprising formulating the artificial blood from:

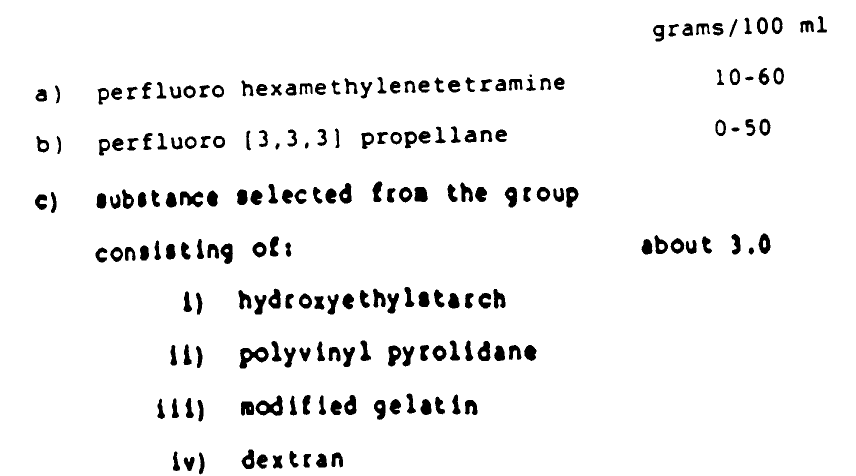

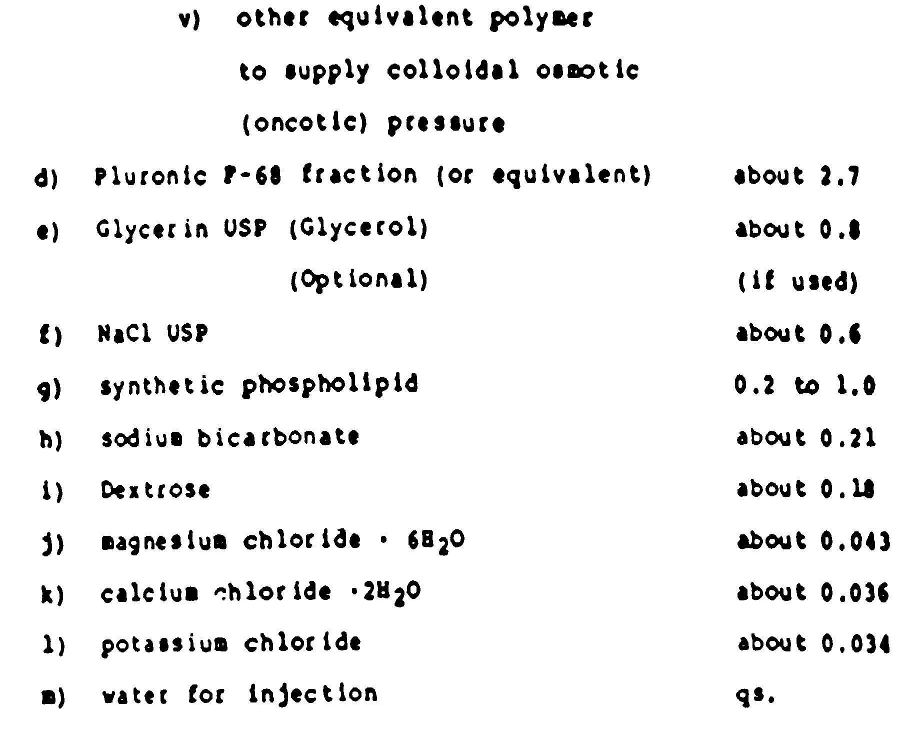

7. Use of material made by the method according to any one of claims 1 to 6 for the manufacture of a medicament for use as an artificial blood.

## Patentansprüche (Patentansprüche für folgende(n) Vertragsstaat(en): BE, CH, DE, FR, GB, IT, LI, LU, NL, SE)

1. Die Verbindung Perfluor-hexamethylentetramin.

2. Die Verbindung des Anspruchs 1 mit der folgenden Struktur:

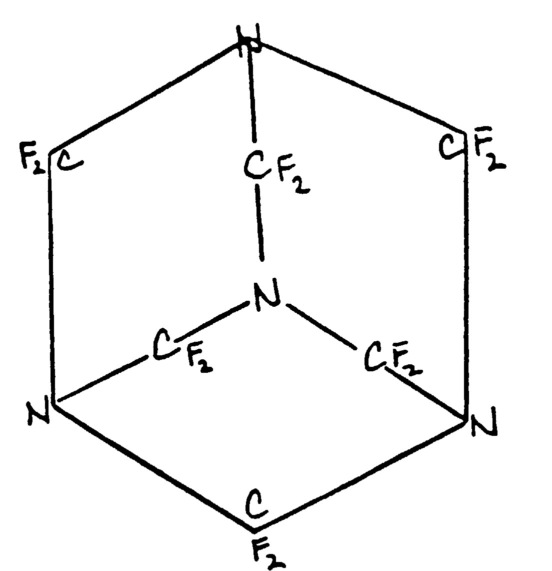

3. Die Verbindung nach Anspruch 2, worin Sauerstoff in der Molekül struktur eingeschlossen ist und die folgende Struktur aufweist:

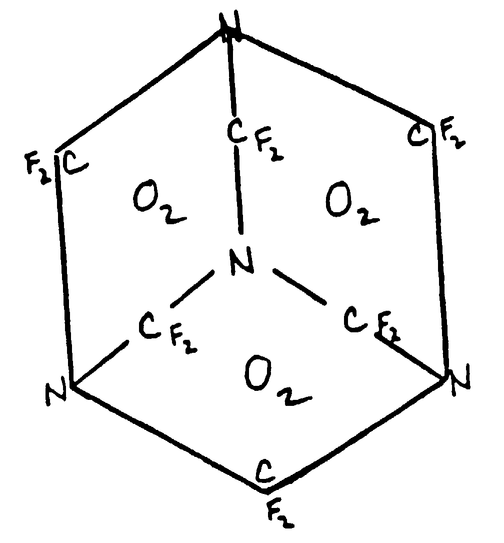

4. Künstliches Blut, umfassend eine Emulsion der Verbindung einer der Ansprüche 1 bis 3 in Wasser, wobei die Emulsion ein nicht toxisches Emulgiermittel und ein synthetisches Phospholipid enthält, welches keine Spur von Eiproteinen enthält.

5. Künstliches Blut nach Anspruch 4, worin das synthetische Phospholipid die folgende Struktur besitzt, 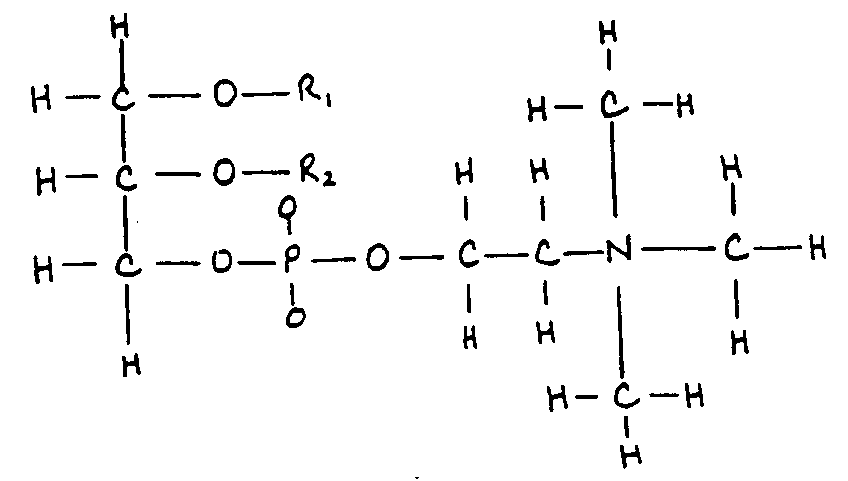 worin R₁ und R₂ Fettsäuren sind.

6. Synthetisches Blut mit der folgenden Zusammensetzung:

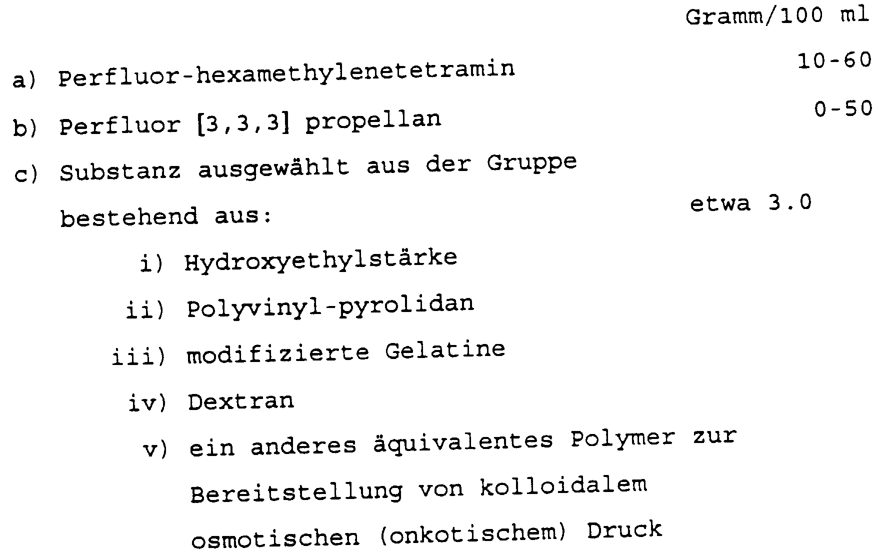

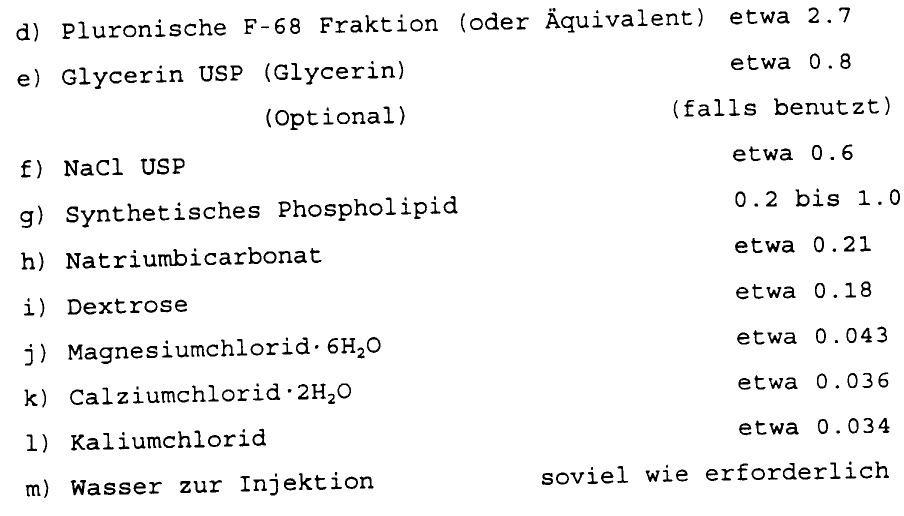

7. Material nach einem der Ansprüche 1 bis 6 zur therapeutischen Anwendung.

8. Anwendung des Materials nach einem der Ansprüche 1 bis 6 zur Herstellung eines Medikaments zur Anwendung als künstliches Blut.

## Patentansprüche (Patentansprüche für folgende(n) Vertragsstaat(en): AT, ES, GR)

1. Verfahren zur Herstellung von künstlichem Blut, umfassend die Herstellung einer Emulsion in Wasser, wobei die Emulsion die Verbindung Perfluor-hexamethylentetramin enthält.

2. Verfahren nach Anspruch 1, worin die Verbindung die folgende Struktur aufweist:

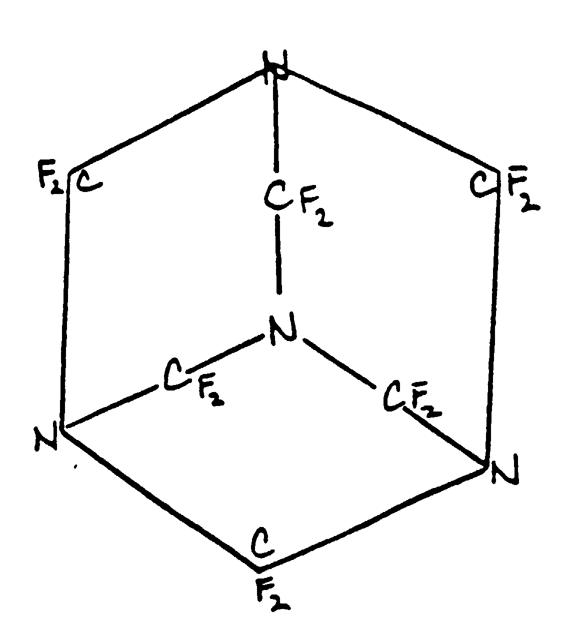

3. Verfahren nach Anspruch 2, worin Sauerstoff in die Molekülstruktur der Verbindung wie folgt eingeschlossen ist:

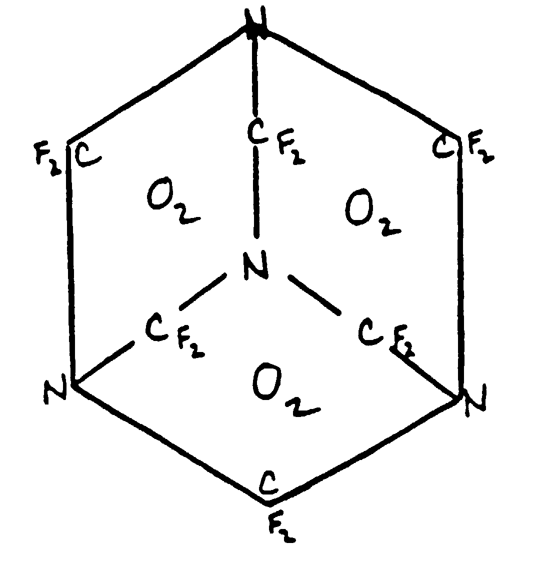

4. Verfahren nach einem der Ansprüche 1 bis 3, worin ein nicht toxisches Emulgiermittel und ein synthetisches Phospholipid, welches keine Spur von Eiproteinen enthält, in der Emulsion enthalten sind.

5. Verfahren nach Anspruch 4, worin das synthetische Phospholipid die folgende Struktur besitzt: 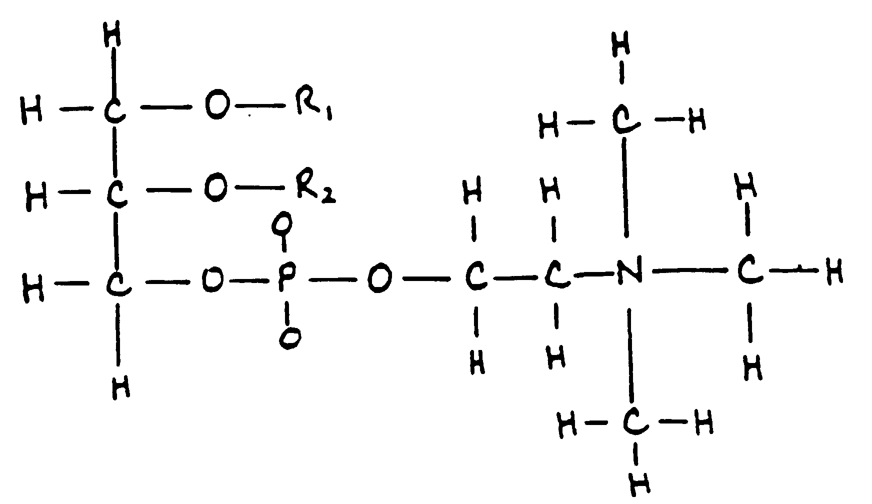 worin R₁ und R₂ Fettsäuren sind.

6. Verfahren nach Anspruch 1, worin das synthetische Blut in der folgenden Formulierung hergestellt wird:

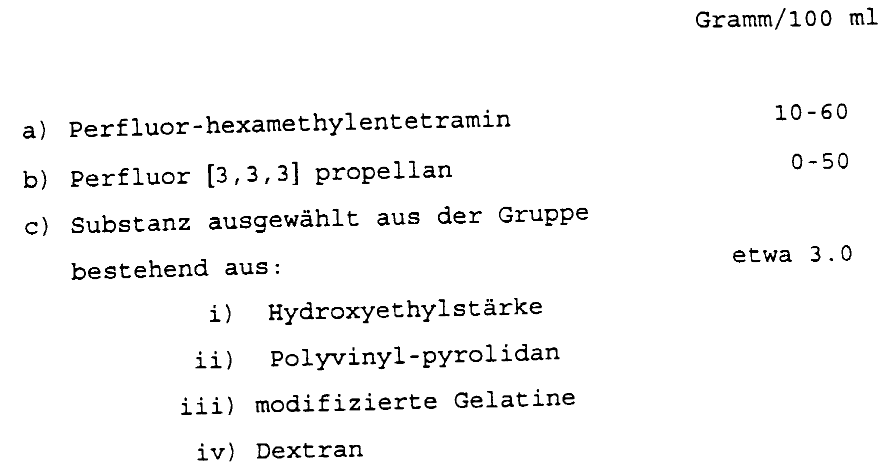

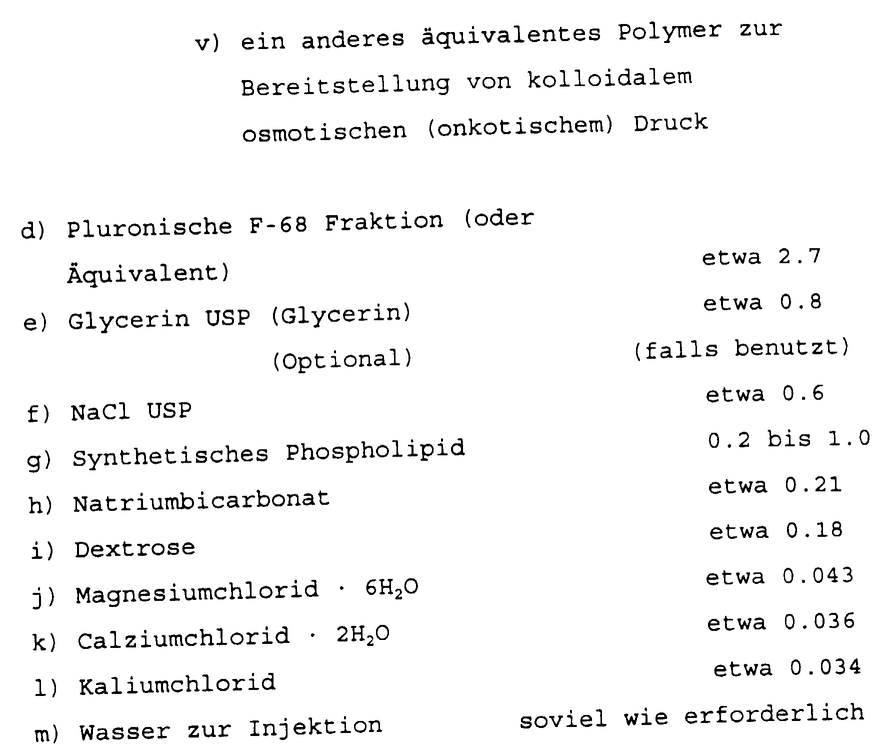

7. Anwendung des Materials nach dem Verfahren gemäß einem der Ansprüche 1 bis 6 zur Herstellung eines Medikaments zur Anwendung als künstliches Blut.

## Revendications (Revendications pour l'(les) Etat(s) contractant(s) suivant(s): BE, CH, DE, FR, GB, IT, LI, LU, NL, SE)

1. La perfluoro-hexaméthylènetétramine.

2. Composé selon la revendication 1, de structure :

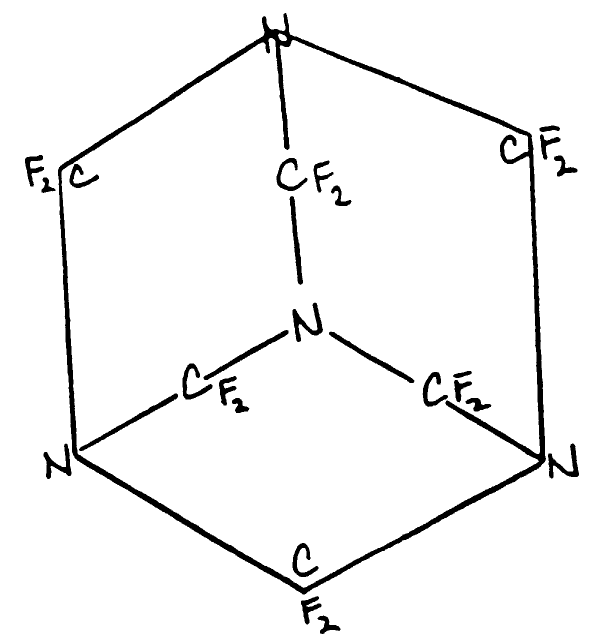

3. Composé selon la revendication 2, dans laquelle de l'oxygène est piégé dans la structure moléculaire, de structure :

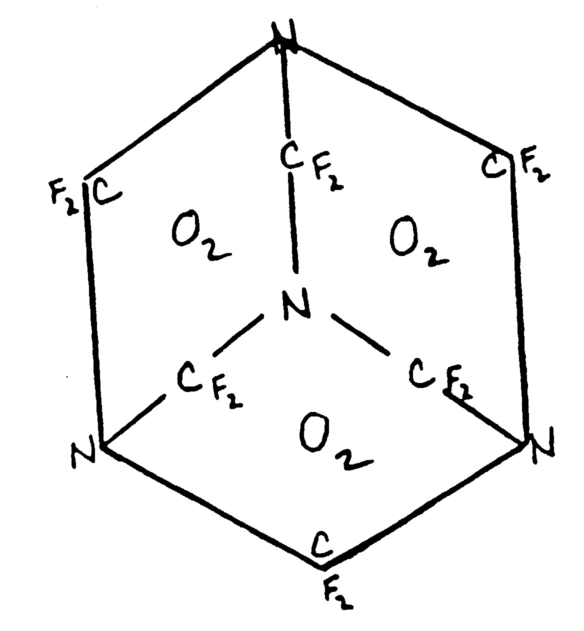

4. Sang artificiel comprenant une émulsion du composé selon l'une quelconque des revendications 1 à 3, dans l'eau, ladite émulsion contenant un émulsifiant non toxique et phospholipide synthétique ne contenant pas de trace de protéine dérivée de l'oeuf.

5. Sang artificiel selon la revendication 4, dans lequel le phospholipide synthétique a pour formule : 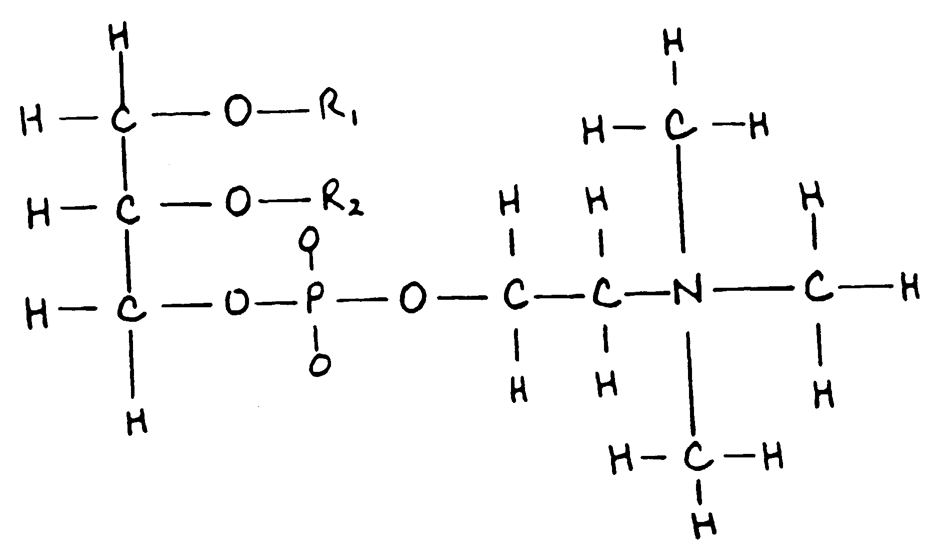 dans laquelle R₁ et R₂ représentent des acides gras.

6. Sang synthérique présentant la composition suivante :

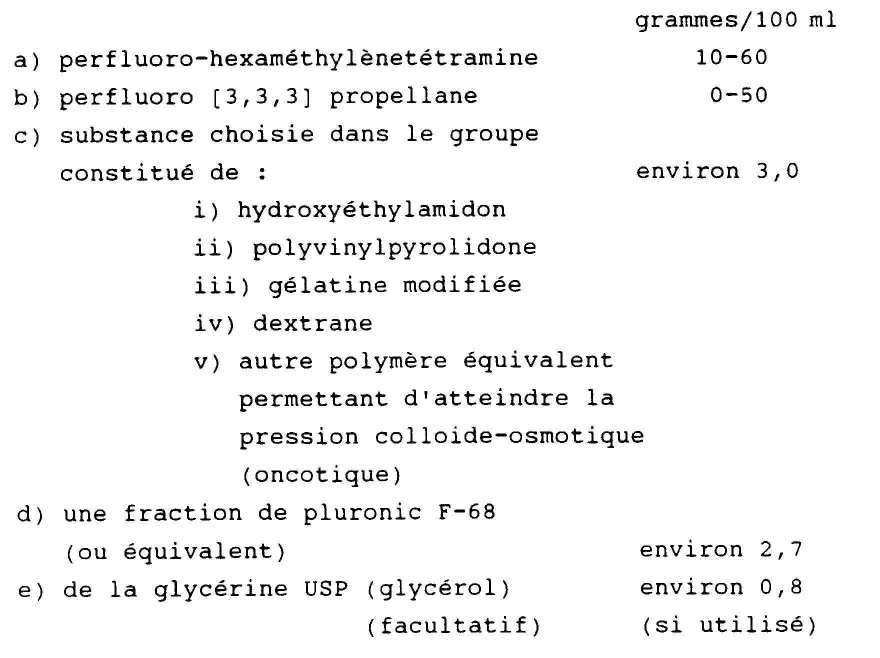

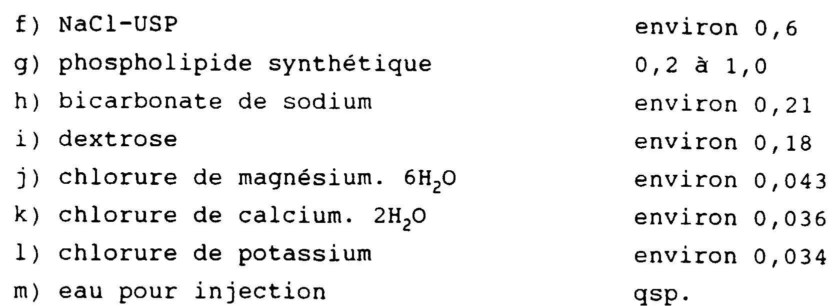

7. Matériau selon l'une quelconque des revendications 1 à 6, pour son utilisation en thérapie.

8. Utilisation d'un matériau selon l'une quelconque des revendications 1 à 6 pour la fabrication d'un médicament destiné à être utilisé en tant que sang artificiel.

## Revendications (Revendications pour l'(les) Etat(s) contractant(s) suivant(s): AT, ES, GR)

1. Procédé pour la préparation d'un sang artificiel comprenant la formation d'une émulsion dans l'eau, ladite émulsion contenant de la perfluoro-hexaméthylènetétramine.

2. Procédé selon la revendication 1, dans lequel la perfluoro-hexaméthylènetétramine a pour formule :

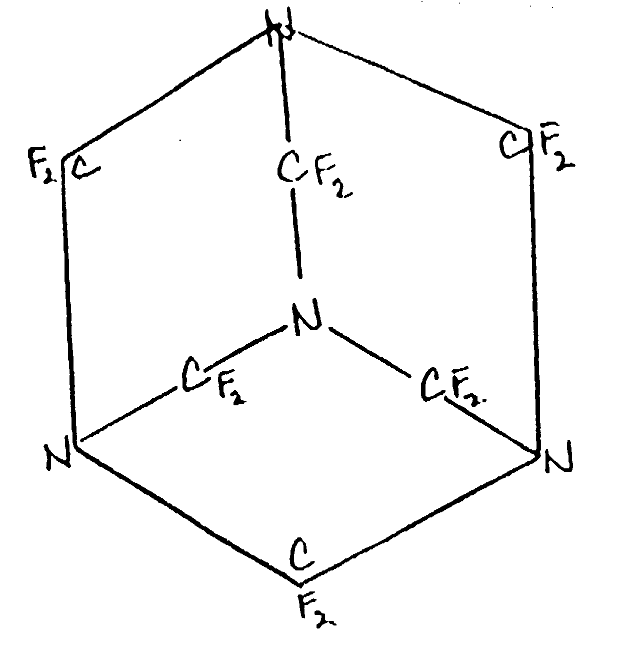

3. Procédé selon la revendication 2, dans lequel de l'oxygène est piégé dans la structure moléculaire de la perfluoro-hexaméthylènetétramine, comme suit :

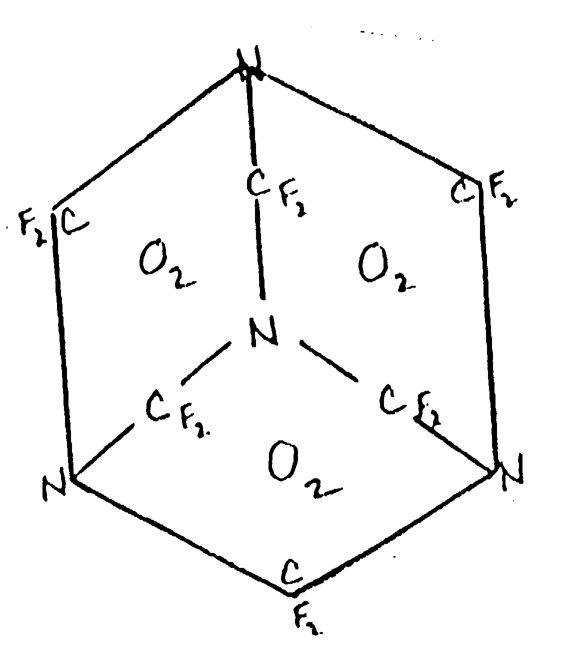

4. Procédé selon l'une quelconque des revendications 1 à 3, dans lequel l'émulsion comprend un émulsifiant non toxique et un phospholipide synthétique ne contenant pas de trace de protéine dérivée de l'oeuf.

5. Procédé selon la revendication 4, dans lequel le phospholipide synthétique a pour formule : 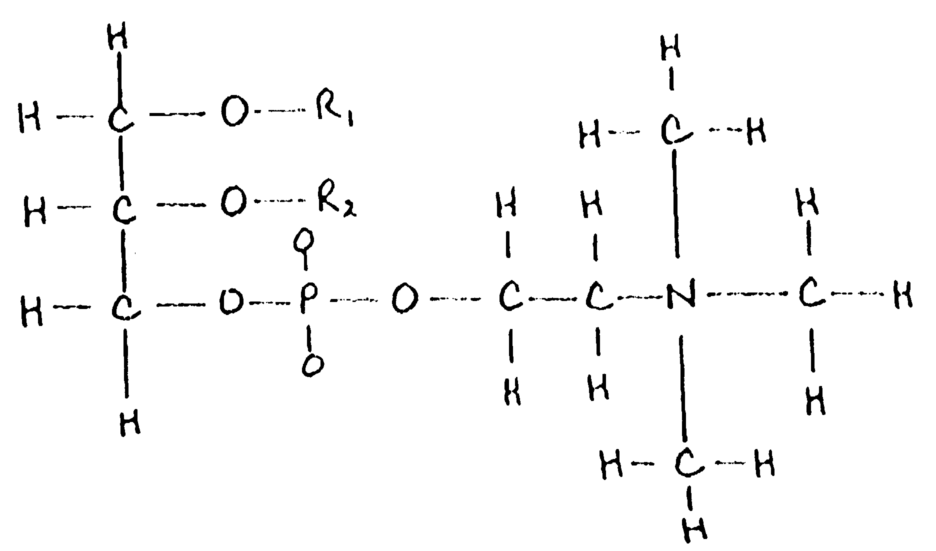 dans laquelle R₁ et R₂ sont des acides gras.

6. Procédé selon la revendication 1 comprenant la formulation du sang artificiel à partir de :

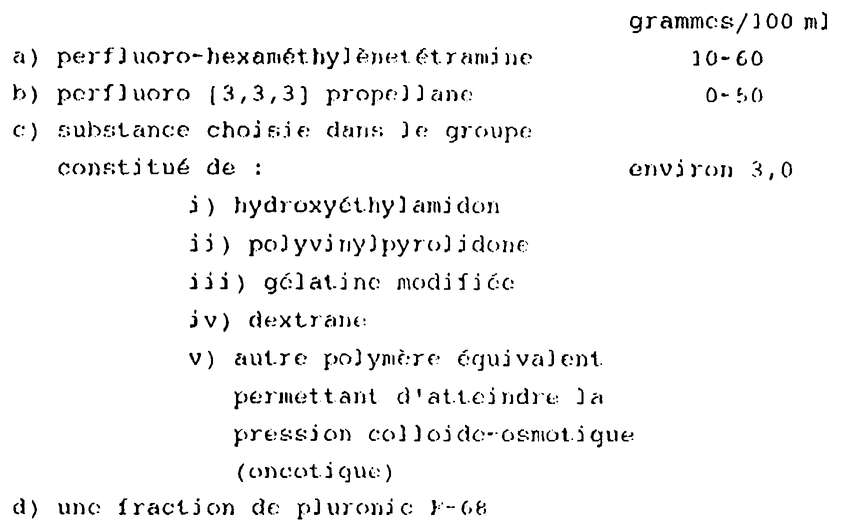

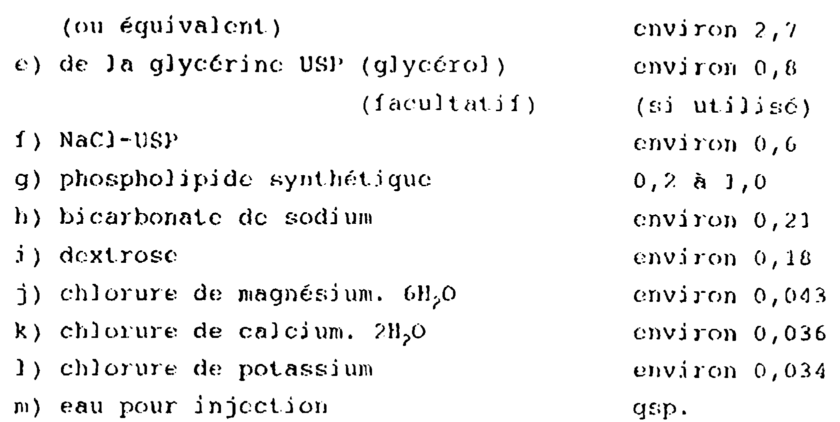

7. Utilisation d'un matériau préparé selon le procédé de l'une quelconque des revendications 1 à 6, pour la fabrication d'un médicament destiné à être utiliser en tant que sang artificiel.
